Europäisches Patentamt

European Patent Office

Office européen des brevets

(11) Veröffentlichungsnummer: **0 304 703 B1**

# EUROPÄISCHE PATENTSCHRIFT

(45) Veröffentlichungstag der Patentschrift: **15.07.92**

(51) Int. Cl.5: **A61B** 17/56, F16B 7/04

(21) Anmeldenummer: **88112768.2**

(22) Anmeldetag: **05.08.88**

(54) Fixateur zum Festlegen von Knochenteilen im Körper.

(30) Priorität: **17.08.87 DE 3727400**

(43) Veröffentlichungstag der Anmeldung:
**01.03.89 Patentblatt 89/09**

(45) Bekanntmachung des Hinweises auf die
Patenterteilung:
**15.07.92 Patentblatt 92/29**

(84) Benannte Vertragsstaaten:
**AT CH ES FR GB IT LI**

(56) Entgegenhaltungen:
**GB-A- 2 033 758**
**US-A- 4 475 843**
**US-A- 4 620 533**

(73) Patentinhaber: **AESCULAP AG**
**Möhringer Strasse 125**
**W-7200 Tuttlingen(DE)**

(72) Erfinder: **Braun, Karl**
**Oberer Brühl 305**
**W-7201 Talheim(DE)**

(74) Vertreter: **Hoeger, Stellrecht & Partner**
**Uhlandstrasse 14 c**
**W-7000 Stuttgart 1(DE)**

Rank Xerox (UK) Business Services

## Beschreibung

Die Erfindung betrifft einen Fixateur zum Festlegen von Knochenteilen im Körper mittels in die Knochenteile einschraubbarer Stangen mit Haltestangen und mit Klemmelementen zum Festklemmen der einschraubbaren Stangen an den Haltestangen oder der Haltestangen aneinander, welche Klemmelemente zwei gegeneinander spannbare Halter umfassen, die je eine Durchstecköffnung für eine Stange aufweisen, wobei sich die Querschnitte der seitlich offenen, in parallel zueinander liegenden Ebenen verlaufenden Durchstecköffnungen bei gegeneinander gespannten Haltern überlappen.

Ein Fixateur mit diesen Merkmalen ist beispielsweise aus der deutschen Patentschrift 28 47 006 bekannt. Mit einem solchen Fixateur können verschiedene Stangen untereinander unter unterschiedlichem Winkel so fest miteinander verbunden werden, daß ein fachwerkartiges Stützgerüst zur Festlegung von Knochenteilen gebildet wird. Damit die Stangen an den Klemmstellen ausreichend festgelegt sind, müssen die beiden Halter eines Klemmelementes mit hohen Kräften gegeneinander gespannt werden. Dies kann beim Anlegen im Einzelfall schwierig sein, beispielsweise wenn die Stelle, an der sich das Klemmelement befindet, nur schwer zugänglich ist. Außerdem besteht die Gefahr, daß bei einer geringfügigen Lockerung der die Halter gegeneinander drückenden Spannkraft die Klemmung zwischen gegeneinander gepreßten Stäben nicht mehr ausreicht, um diese in ihrer Relativposition genügend zu fixieren.

Es ist Aufgabe der Erfindung, einen gattungsgemäßen Fixateur derart zu verbessern, daß auch bei Verwendung geringerer Klemmkräfte eine einwandfreie und in allen Fällen ausreichende Festlegung der aneinander gedrückten Stangen erreichbar ist.

Diese Aufgabe wird bei einem Fixateur der eingangs beschriebenen Art erfindungsgemäß dadurch gelöst, daß die Stangen zumindest in dem in den Durchstecköffnungen angeordneten Bereich in Umfangsrichtung umlaufende, im Querschnitt nach außen spitz zulaufende Rippen tragen. Beim Spannen der Klemmelemente werden die Stangen mit den Rippen gegeneinander gedrückt, wobei sich die spitz zulaufenden Rippen der beiden gegeneinander gedrückten Stangen ineinander drücken und teilweise unter Verformung dieser spitz zulaufenden Rippen miteinander verzahnen. Dadurch wird zusätzlich zu dem reinen Kraftschluß eine formschlüssige Komponente für die Klemmverbindung hinzugefügt, die die Festlegung der beiden Stangen in der einmal eingenommenen Relativposition gegenüber einer reinen Klemmung wesentlich verbessert.

Bei einem bevorzugten Ausführungsbeispiel können die Rippen beispielsweise durch die Gänge eines auf den Stangen angeordneten Außengewindes gebildet sein.

Im allgemeinen Fall werden die Durchstecköffnungen der beiden Halter eines Klemmelementes miteinander einen Winkel einschliessen, vorzugsweise verlaufen die Durchstecköffnungen dabei senkrecht zueinander. Durch diesen Winkel ergibt sich eine besonders günstige Verzahnung der Rippen der beiden aneinanderliegenden Stangen, da jede Rippe der einen Stange an mehreren Rippen der anderen Stange anliegt und in diese einschneidet. Dadurch ergeben sich eine Vielzahl von im Abstand zueinander liegenden Eingriffspunkte, die eine Festlegung der beiden Stangen gegen eine Drehung oder Längsverschiebung gewährleisten.

Es kann dabei vorgesehen sein, daß die beiden Halter mittels eines senkrecht zur Trennebene der beiden Halter angeordneten Schraubbolzens und einer auf diesen aufgeschraubten Mutter gegeneinander gespannt sind. Eine solche Anordnung ermöglicht es, die beiden Halter um die Längsachse des Schraubbolzens gegeneinander zu verdrehen, so daß auch die Winkel der gegeneinander geklemmten Stangen dadurch veränderbar sind, solange die Halter nicht gegeneinander gespannt sind.

Besonders vorteilhaft ist es dabei, wenn die Mutter an einer Endfläche einen über die ebenen Seitenflächen überstehenden Bund trägt. Wenn dieser Bund an der dem Halter gegenüberliegenden Seite angeordnet wird, verhindert der Bund ein Abgleiten eines auf die Mutter aufgesetzten Schlüssels, so daß das Spannen der beiden Halter erheblich begünstigt wird.

Eine weitere vorteilhafte Ausgestaltung ergibt sich dann, wenn die Außenkante des Bundes gerändelt ist. Dies ermöglicht es, die Mutter auch von Hand relativ fest auf den Schraubbolzen aufzuschrauben, wobei es besonders vorteilhaft ist, daß der schmale, gerändelte Bund über die Seitenflächen der Mutter vorsteht, so daß der schmale Bund mit den Fingern besonders effektiv ergriffen werden kann.

Die nachfolgende Beschreibung einer bevorzugten Ausführungsform der Erfindung dient im Zusammenhang mit der Zeichnung der näheren Erläuterung. Es zeigen:

Figur 1     eine Seitenansicht eines fest gespannten Klemmelementes eines Fixateurs mit zwei eingespannten Stangen und

Figur 2     eine Draufsicht auf das Klemmelement der Figur 1.

In der Zeichnung sind von einem Fixateur lediglich zwei gegeneinander geklemmte Stangen 1 und 2 in ihrem Klemmbereich und ein Klemmelement 3 dargestellt. Diese Stangen und Klemmelemente können als Teil eines Fixateurs zum Positionieren von Knochenteilen verwendet werden, wie dies aus der deutschen Patentschrift 28 47 006 an sich bekannt ist. Aus diesem Grunde wird der Gesamtaufbau des Fixateurs daher nachstehend nicht mehr ausführlich beschrieben. Das Klemmelement umfaßt zwei im wesentlichen quaderförmige Halter 4 und 5. Der eine Halter ist einstückig mit einem senkrecht aus einer Seitenfläche hervorstehenden Gewindebolzen 6 verbunden, der durch eine Durchgangsbohrung 7 im anderen Halter hindurchgesteckt ist. Auf das Ende des Gewindebolzens 6 ist eine Mutter 8 aufgeschraubt, die dadurch die beiden Halter 4 und 5 längs der einander zugewandten Trennflächen 9 bzw. 10 gegeneinander spannt.

Die Mutter 8 ist eine normale Sechskantmutter, die an ihrer dem Klemmelement 3 abgewandten Seite einen über die ebenen Seitenflächen 11 der Mutter 8 hervorstehenden, im Querschnitt kreisförmigen Bund 12 trägt, dessen Außenkante 13 gerändelt ist. Durch diesen über die Seitenflächen 11 vorstehenden Bund 12 wird ein seitliches Abrutschen eines auf die Mutter 8 aufgesetzten Steckschlüssels verhindert, außerdem kann mittels des gerändelten Bundes 12 die Mutter 8 von Hand auf dem Gewindebolzen 6 verdreht werden.

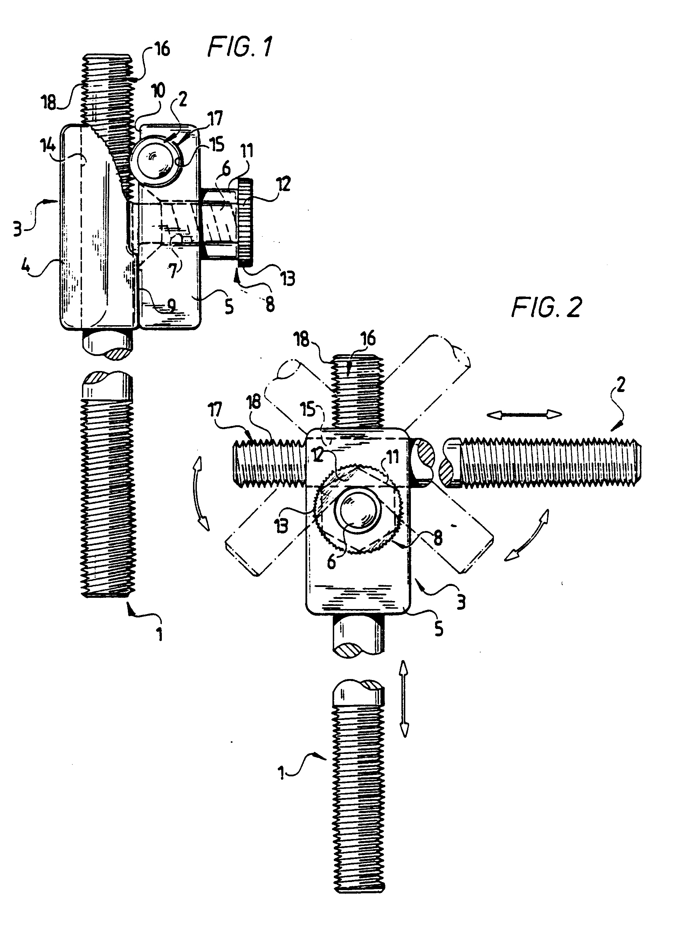

In jedem der beiden Halter 4 und 5 befindet sich eine parallel zur Trennfläche 9 bzw. 10 verlaufende Durchstecköffnung 14 bzw. 15, die zumindesten über einen Teil ihrer Länge seitlich offen ist, und zwar auf der dem jeweils anderen Halter zugewandten Seite. Die Durchstecköffnungen 14 und 15 sind dabei so angeordnet, daß sich die kreisförmigen Querschnitte der Durchstecköffnungen 14 und 15 geringfügig überlappen, wenn die beiden Halter 4 und 5 gegeneinander gespannt sind (Figur 1).

Die in die Durchstecköffnungen 14 und 15 eingesteckten Stangen 1 bzw. 2 tragen jeweils ein Außengewinde 16 bzw. 17, so daß sie in dem in die Durchstecköffnungen eingesteckten Bereich eine Vielzahl von in Umfangsrichtung umlaufenden, nach außen spitz zulaufenden Rippen 18 aufweisen.

Vor dem Festspannen der Mutter 8 sind die beiden Halter 4 und 5 mit den in sie eingesetzten Stangen 1 und 2 um die Längsachse des Gewindebolzens 6 gegeneinander verdrehbar, so daß auch der Winkel zwischen den beiden Stangen 1 und 2 beliebig einstellbar ist. Dies ist in Figur 2 dargestellt, in der die Stangen 1 und 2 in einer rechtwinkligen Anordnung mit ausgezogenen Linien, in anderen Winkellagen dagegen in strichpunktierten Linien wiedergegeben sind. Wenn die Stangen 1 und 2 in die gewünschte Orientierung verdreht sind und wenn sie auch die gewünschte Einstecktiefe in den Durchstecköffnungen 14 und 15 einnehmen, werden die beiden Halter durch Spannen der Mutter 8 gegeneinander gedrückt. Dadurch legen sich die Rippen 18 der beiden Stangen 1 und 2 in den Teil, in dem sie durch die seitlich offenen Durchstecköffnungen 14 und 15 hervorstehen, gegeneinander und graben sich in die Rippen der jeweils anderen Stange ein, wobei jede Rippe normalerweise Kontakt mit mehreren Rippen der jeweils anderen Stange hat. Dadurch ergibt sich eine Verzahnung der beiden Stangen in der jeweils eingenommenen Lage, die durch lokale Verformungen an einer Vielzahl von Stellen entsteht und auf diese Weise eine Fixierung der Relativposition der beiden Stangen 1 und 2 sichert. Diese Fixierung ist dabei wesentlich wirkungsvoller als bei zwei Stangen mit glatter Außenfläche, da durch das Vorsehen der Rippen 18 die effektive Berührungsfläche erheblich vergrößert wird.

In dem dargestellten Ausführungsbeispiel werden die Rippen 18 durch Außengewinde 16 und 17 erzeugt, es ist aber auch möglich, diese Rippen in anderer Weise herzustellen, beispielsweise durch Einschneiden von benachbarten Nuten in die Außenfläche der Stangen.

## Patentansprüche

1. Fixateur zum Festlegen von Knochenteilen im Körper mittels in die Knochenteile einschraubbarer Stangen (1, 2) mit Haltestangen und mit Klemmelementen (3) zum Festklemmen der einschraubbaren Stangen (1, 2) an den Haltestangen oder der Haltestangen aneinander, welche Klemmelemente zwei gegeneinander spannbare Halter (4, 5) umfassen, die je eine Durchstecköffnung (14, 15) für eine Stange aufweisen, wobei sich die Querschnitte der seitlich offenen, in parallel zueinander liegenden Ebenen (9, 10) verlaufenden Durchstecköffnungen bei gegeneinander gespannten Haltern überlappen, **dadurch gekennzeichnet,** daß die Stangen (1, 2) zumindest in dem in den Durchstecköffnungen (14, 15) angeordneten Bereich in Umfangsrichtung umlaufende, im Querschnitt nach außen spitz zulaufende Rippen (18) tragen.

2. Fixateur nach Anspruch 1, dadurch gekennzeichnet, daß die Rippen (18) durch die Gänge eines auf den Stangen (1, 2) angeordneten Außengewindes (16 bzw. 17) gebildet sind.

3. Fixateur nach einem der voranstehenden Ansprüche, dadurch gekennzeichnet, daß die Durchstecköffnungen (14, 15) der beiden Halter (4 bzw. 5) miteinander einen Winkel einschließen.

4. Fixateur nach Anspruch 3, dadurch gekennzeichnet, daß die Durchstecköffnungen (14, 15) der beiden Halter (4 bzw. 5) etwa senkrecht zueinander verlaufen.

5. Fixateur nach einem der voranstehenden Ansprüche, dadurch gekennzeichnet, daß die beiden Halter (4, 5) mittels eines senkrecht zur Trennebene der beiden Halter (4, 5) angeordneten Schraubbolzens (6) und eine darauf geschraubte Mutter (8) gegeneinander gespannt sind.

6. Fixateur nach Anspruch 5, dadurch gekennzeichnet, daß die Mutter (8) an einer Endfläche einen über die ebenen Seitenflächen (11) überstehenden Bund (12) trägt.

7. Fixateur nach Anspruch 6, dadurch gekennzeichnet, daß die Außenkante (13) des Bundes (12) gerändelt ist.

## Claims

1. A fixator for holding bone sections in the body by means of bars (1, 2) which can be screwed into the bone sections, with supporting bars and clamping elements (3) for clamping the screw-in bars (1, 2) to the supporting bars or for clamping the supporting bars to one another, which clamping elements comprise two supports (4, 5) which can be tensioned against one another and which each have an insertion opening (14, 15) for a bar, the cross-sections of the insertion openings, which are open at the sides and which extend in parallel planes (9, 10), overlapping when the supports are tensioned against one another, characterised in that the bars (1, 2) carry ribs (18) at least in the region arranged in the insertion openings (14, 15), these ribs encircling in the peripheral direction and being pointed outwards in cross-section.

2. A fixator according to Claim 1, characterised in that the ribs (18) are formed by the threads of an external screw thread (16, 17) arranged on the bars (1 and 2 respectively).

3. A fixator according to any one of the preceding Claims, characterised in that the insertion openings (14, 15) of the two supports (4 and 5 respectively) together form an angle.

4. A fixator according to Claim 3, characterised in that the insertion openings (14, 15) of the two supports (4 and 5 respectively) extend substantially normal to one another.

5. A fixator according to any one of the preceding Claims, characterised in that the two supports (4, 5) are tensioned against one another by means of a screw bolt (6), arranged normally in relation to the separating plane of the two supports (4, 5), and a nut (8) screwed thereon.

6. A fixator according to Claim 5, characterised in that, on one end face, the nut (8) carries a collar (12) which projects over the flat side faces (11).

7. A fixator according to Claim 6, characterised in that the outside edge (13) of the collar (12) is knurled.

## Revendications

1. Fixation pour fixer des fragments d'os dans le corps au moyen de broches (1, 2) qui peuvent se visser dans les fragments d'os et présentent des broches de maintien et des éléments de bridage (3) pour bloquer les broches (1, 2), qui peuvent se visser, contre les broches de maintien ou les broches de maintien l'une contre l'autre, les éléments de bridage comprenant deux supports (4, 5) qui peuvent se serrer l'un contre l'autre et qui présentent chacun une ouverture d'insertion (14, 15) pour une broche, étant précisé que, lorsque les supports sont serrés l'un contre l'autre, les sections des ouvertures d'insertion, ouvertes latéralement et définies par des plans (9, 10) parallèles l'un à l'autre, se recouvrent, fixation caractérisée par le fait qu'au moins dans la zone disposée dans les ouvertures d'insertion (14, 15), les broches portent des nervures (18) qui tournent dans la direction périphérique et se terminent en pointe vers l'extérieur en coupe.

2. Fixation selon la revendication 1, caractérisée par le fait que les nervures (18) sont formées par les filets d'un filetage extérieur (16 ou 17) disposé sur les broches (1, 2).

3. Fixation selon l'une des revendications précédentes, caractérisée par le fait que les ouvertures d'insertion (14, 15) des deux supports (4 ou 5) forment un angle entre elles.

4. Fixation selon la revendication 3, caractérisée par le fait que les ouvertures d'insertion (14, 15) des deux supports (4 ou 5) sont orientées à peu près perpendiculairement l'une par rapport à l'autre.

5. Fixation selon l'une des revendications précédentes, caractérisée par le fait que les deux supports (4, 5) sont serrés l'un contre l'autre au moyen d'une tige filetée (6), disposée perpendiculairement au plan de joint des deux supports (4, 5) et d'un écrou (8) vissé sur elle.

6. Fixation selon la revendication 5, caractérisée par le fait que l'écrou (8) porte, à sa surface d'extrémité, un collet qui déborde au-delà des surfaces latérales lisses (11).

7. Fixation selon la revendication 6, caractérisée par le fait que la bordure extérieure (13) du collet (12) est moletée.

FIG. 1

FIG. 2